(19) Europäisches Patentamt
European Patent Office
Office européen des brevets

(11) EP 3 590 511 A1

(12) EUROPEAN PATENT APPLICATION
published in accordance with Art. 153(4) EPC

(43) Date of publication:
**08.01.2020 Bulletin 2020/02**

(21) Application number: **18760692.6**

(22) Date of filing: **28.02.2018**

(51) Int Cl.:
***A61K 31/403*** (2006.01) ***A61P 25/32*** (2006.01)

(86) International application number:
**PCT/JP2018/007628**

(87) International publication number:
**WO 2018/159716 (07.09.2018 Gazette 2018/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **02.03.2017 JP 2017039106**

(71) Applicants:
- **Sanwa Kagaku Kenkyusho Co., Ltd**
**Nagoya-shi, Aichi 461-8631 (JP)**
- **UBE Industries, Ltd.**
**Yamaguchi 755-8633 (JP)**

(72) Inventors:
- **NAKAJIMA, Atsushi**
**Nagoya-shi**
**Aichi 461-8631 (JP)**
- **YAMASHITA, Tokuyuki**
**Nagoya-shi**
**Aichi 461-8631 (JP)**

(74) Representative: **Grünecker Patent- und Rechtsanwälte**
**PartG mbB**
**Leopoldstraße 4**
**80802 München (DE)**

# (54) THERAPEUTIC AGENT FOR ALCOHOL USE DISORDERS

(57) An object he present invention is to provide a medicament for therapy of alcohol use disorder. The present invention relates to a medicament for therapy of alcohol use disorder, containing a compound represented by the following general formula (I) or a pharmacologically acceptable salt thereof as an active ingredient:

[Chemical Formula 1]

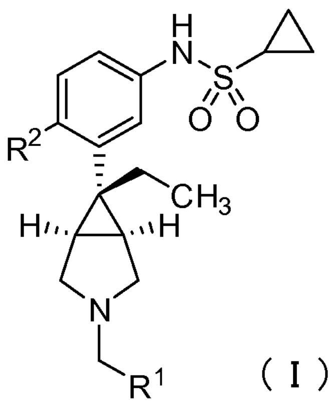

(I)

wherein $R^2$ represents a hydrogen atom or a halogen atom and $R^1$ represents a group selected from the group consisting of

EP 3 590 511 A1

[Chemical Formula 2]

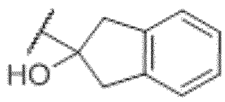

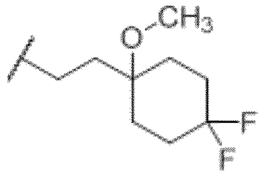

and

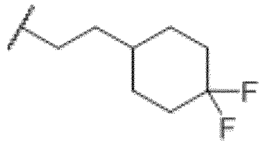

**Description**

TECHNICAL FIELD

The present invention relates to a medicament for therapy of alcohol use disorder, containing a 3-azabicyclo[3.1.0]hexane derivative with μ opioid receptor antagonist activity as an active ingredient.

BACKGROUND ART

Opioid is a collective name of alkaloids such as narcotic analgesics and related synthetic analgesics and synthetic or endogenous peptides having morphine-like activity. There are currently four known subtypes of opioid receptors involved in onset of action of opioids: μ, κ, δ and ORL-1. Among these, μ opioid receptors are the receptors that are most related to the action of morphine, and in addition to morphine, fentanyl and endogenous opioids, methionine enkephalin and β-endorphin, also act on the receptors.
μ opioid receptor agonists, morphine and fentanyl, are known to produce reward effects or euphoria, and are also known to form dependence after repeated use. It is also believed that an endogenous opioid, β-endorphin, and μ opioid receptors play an important role in an effect of enhancing alcohol reward.
For therapy of alcohol use disorder, μ opioid receptor antagonists such as naltrexone are used. However, side effects such as nausea, vomiting, hyperalgesia such as abdominal pain and diarrhea have been observed and thus use thereof has not always been satisfactory (Non Patent Literature 1). Therefore, there is an expectation for development of a therapeutic agent for alcohol use disorder with less side effects.
So far, many documents (Patent Literatures 1 to 3 and Non Patent Literature 2) have reported actions and effects of 3-azabicyclo[3.1.0]hexane derivatives with μ opioid receptor antagonist activity on alcohol use disorder. However, the compounds disclosed in the documents have different structures from the compound (Patent Literature 4) according to the invention of the present application. It is not known how the compound of the invention of the present application affects on spontaneous ethanol intake or whether or not the compound of the invention of the present application has a therapeutic effect on alcohol use disorder.

CITATION LISTS

PATENT LITERATURE

Patent Literature 1: WO 2000/039089
Patent Literature 2: WO 2001/098267
Patent Literature 3: WO 2003/035622
Patent Literature 4: WO 2015/076310

NON-PATENT LITERATURE

Non Patent Literature 1: Drugs, 35, 192-213 (1988)
Non Patent Literature2: Medicinal Chemistry Communications, 2 (2011) 1001-1005

SUMMARY OF INVENTION

TECHNICAL PROBLEMS

An object of the present invention is to provide a novel medicament for therapy of alcohol use disorder.

SOLUTIONS TO PROBLEMS

Inventors of the present invention have carried out researches in order to attain the object and, as a result, found that the following 3-azabicyclo(3.1.0)hexane derivative is useful for therapy of alcohol use disorder, thereby completing the present invention.
Thus, the present invention is as follows:

<1> A medicament for therapy of alcohol use disorder, containing a compound represented by a general formula (I) below or a pharmacologically acceptable salt thereof as an active ingredient:

[Chemical Formula 1]

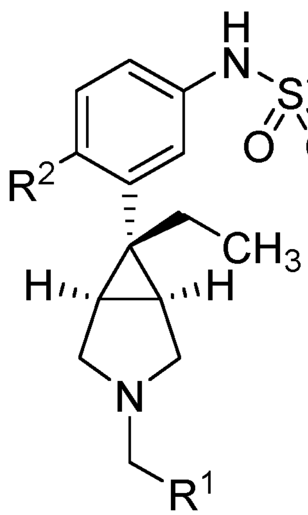

( I )

wherein $R^2$ represents a hydrogen atom or a halogen atom, and $R^1$ represents a group selected from the group consisting of

[Chemical Formula 2]

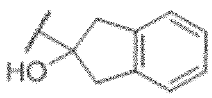

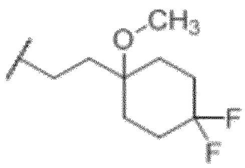

and

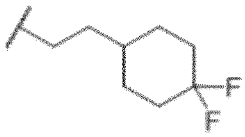

.

<2> The medicament according to <1>, wherein the active ingredient is a compound or a pharmacologically acceptable salt thereof, selected from the group consisting of: N-(3-{(1R,5S,6r)-6-ethyl-3-[(2-hydroxy-2,3-dihydro-1H-inden-2-yl)methyl]-3-azabicyclo[3.1.0]hexan-6-yl}-4-fluorophenyl)cyclopropanesulfonamide, N-(3-{(1R,5S,6r)-3-[3-(4,4-difluoro-1-methoxycyclohexyl)propyl]-6-ethyl-3-azabicyclo[3.1.0]hexan-6-yl}phenyl)cyclopropanesulfonamide, and, N-(3-{(1R,5S,6r)-3-[3-(4,4-difluorocyclohexyl)propyl]-6-ethyl-3-azabicyclo[3.1.0]hexan-6-yl}phenyl)cyclopropanesulfonamide.

<3> The medicament according to <2>, wherein the active ingredient is N-(3-{(1R,5S,6r)-6-ethyl-3-[(2-hydroxy-2,3-dihydro-1H-inden-2-yl)methyl]-3-azabicyclo[3.1.0]hexan-6-yl}-4-fluorophenyl)cyclopropanesulfonamide or a pharmacologically acceptable salt thereof.

<4> The medicament according to <2>, wherein the active ingredient is N-(3-{(1R,5S,6r)-3-[3-(4,4-difluoro-1-methoxycyclohexyl)propyl]-6-ethyl-3-azabicyclo[3.1.0]hexan-6-yl}phenyl)cyclopropanesulfonamide or a pharmacologically acceptable salt thereof.

<5> The medicament according to <2>, wherein the active ingredient is N-(3-{(1R,5S,6r)-3-[3-(4,4-difluorocyclohexyl)propyl]-6-ethyl-3-azabicyclo[3.1.0]hexan-6-yl}phenyl)cyclopropanesulfonamide or a pharmacologically acceptable salt thereof.

<6> The medicament according to <1>, wherein the alcohol use disorder is alcoholism.

<7> The medicament according to <1>, wherein the alcohol use disorder is harmful use of alcohol.

<8> The medicament according to <1>, wherein the alcohol use disorder is alcohol abuse.

<9> The medicament according to <1>, wherein the alcohol use disorder is pre-alcoholism.

ADVANTAGEOUS EFFECTS OF INVENTION

The medicament of the present invention has an effect of suppression of spontaneous ethanol intake. Therefore, the medicament of the present invention is efficacious for therapy of alcohol use disorder and may be used for therapy of alcohol abuse and alcoholism.

BRIEF DESCRIPTION OF DRAWINGS

Fig. 1 illustrates pure ethanol consumption per 2 hours in alcohol-addicted rats to which vehicle or test substance A is subcutaneously administered.
Fig. 2 illustrates pure ethanol consumption per 2 hours in alcohol-addicted rats to which vehicle or test substance B is subcutaneously administered.
Fig. 3 illustrates pure ethanol consumption per 2 hours in alcohol-addicted rats to which vehicle or test substance C is subcutaneously administered.
Fig. 4 illustrates pure ethanol consumption per 2 hours in alcohol-addicted rats to which vehicle or test substance A, B or C is subcutaneously administered.

DESCRIPTION OF EMBODIMENTS

The present invention will be hereinafter more specifically described.
The medicament for therapy of alcohol use disorder of the present invention contains a compound represented by the following general formula (I) or a salt thereof as an active ingredient. Among them, N-(3-{(1R,5S,6r)-3-[3-(4,4-difluoro-1-methoxycyclohexyl)propyl]-6-ethyl-3-azabicyclo[3.1.0]hexan-6-yl}phenyl)cyclopropanesulfonamide (compound 1), N-(3-{(1R,5S,6r)-3-[3-(4,4-difluorocyclohexyl)propyl]-6-ethyl-3-azabicyclo[3.1.0]hexan-6-yl}phenyl)cyclopropanesulfonamide (compound 2) and N-(3-{(1R,5S,6r)-6-ethyl-3-[(2-hydroxy-2,3-dihydro-1H-inden-2-yl)methyl]-3-azabicyclo[3.1.0]hexan-6-yl}-4-fluorophenyl)cyclopropanesulfonamide (compound 3) are preferred. The compounds are disclosed in Patent Literature 4 (WO 2015/076310), and compound 1 corresponds to the compound of Example 6 therein, compound 2 corresponds to the compound of Example 4 therein, and compound 3 corresponds to the compound of Example 2 therein, and may be produced according to the production methods in corresponding Examples.

[Chemical Formula 3]

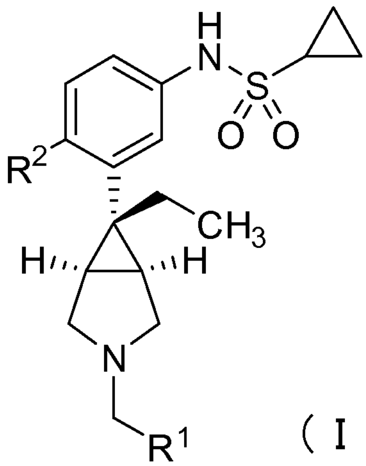

(I)

wherein $R^2$ represents a hydrogen atom or a halogen atom and $R^1$ represents a group selected from the group consisting of

[Chemical Formula 4]

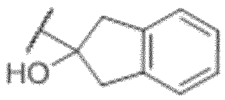 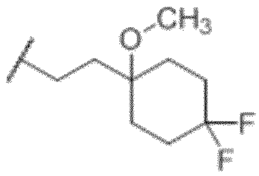 and 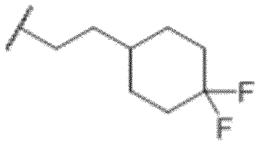.

When the compound forms a salt, the salt is preferably a pharmacologically acceptable salt, and examples thereof include a salt with an inorganic acid, a salt with an organic salt, a salt with a basic or acidic amino acid, and the like. Suitable examples of the salt with an inorganic acid include salts with hydrochloric acid, hydrobromic acid, nitric acid, sulfuric acid, phosphoric acid, and the like. Suitable examples of the salt with an organic acid include salts with acetic acid, trifluoroacetic acid, fumaric acid, oxalic acid, tartaric acid, maleic acid, citric acid, succinic acid, malic acid, methanesulfonic acid, benzoic acid, toluenesulfonic acid, and the like. Suitable examples of the salt with a basic amino acid include salts with arginine and the like. Suitable examples of the salt with an acidic amino acid include salts with aspartic acid, glutamic acid, and the like.

The medicament of the present invention is efficacious for alcohol use disorder. That is, the medicament of the present invention is applicable to a medicament for therapy of alcohol use disorder. The term "alcohol use disorder" as used herein refers to alcoholism, and a condition with mental or physical disorder caused by drinking although it has not been yet developed to alcoholism (harmful use of alcohol), a condition with social or family-related issues developed (alcohol abuse), a condition with alcohol-related issues despite no experience of withdrawal symptom or continuous drinking (pre-alcoholism) and the like.

The dosage form of the medicament of the present invention may be selected according to purposes from various dosage forms described in General Rules for Preparations in "Japanese Pharmacopoeia". For example, in order to form into tablets for oral administration, tablets may be generally formed by selecting additives that are used in the art and can be orally administered. Examples of the additives include various additives that are generally used in the field of drug formulations including excipients such as lactose, crystalline cellulose, white soft sugar and potassium phosphate, a binder, a disintegrating agent, a lubricant and an aggregation preventing agent.

The amount of the compound contained in the medicament of the present invention as an active ingredient is not particularly limited and is appropriately selected from a wide range. The dosage of the compound as an active ingredient is appropriately decided according to the dose regimen thereof, age, sex and other factors of patients or severity of the disease. In case of oral administration, the daily dose of the compound as an active ingredient is 1 $\mu$g to 20 mg, and preferably 10 $\mu$g to 2 mg per kg of body weight, which dose may be appropriately divided and administered 1 to 4 times a day. However, the dosage and frequency of administration is decided in the light of related circumstances including severity of the symptom to be treated, the selected compound to be administered and the selected administration route, and thus the ranges of the dosage and frequency do not limit the scope of the present invention.

## EXAMPLES

The present invention will be further described hereinafter by way of Examples, but the invention is not limited to Examples.

As typical examples of the 3-azabicyclo[3.1.0]hexane derivative, the following compounds were subjected to the pharmacological test.

(Compound 1: test substance A) N-(3-{(1R,5S,6r)-3-[3-(4,4-difluoro-1-methoxycyclohexyl)propyl]-6-ethyl-3-azabicyclo[3.1.0]hexan-6-yl}phenyl)cyclopropanesulfonamide hydrochloride

(Compound 2: test substance B) N-(3-{(1R,5S,6r)-3-[3-(4,4-difluorocyclohexyl)propyl]-6-ethyl-3-azabicyclo[3.1.0]hexan-6-yl}phenyl)cyclopropanesulfonamide hydrochloride

(Compound 3: test substance C) N-(3-{(1R,5S,6r)-6-ethyl-3-[(2-hydroxy-2,3-dihydro-1H-inden-2-yl)methyl]-3-azabicyclo[3.1.0]hexan-6-yl}-4-fluorophenyl)cyclopropanesulfonamide hydrochloride

The test example using the above test substances A to C will be indicated below.

[Pharmacological test example]

Evaluation of effect of suppression of spontaneous ethanol intake

In the experiment, alcohol-addicted rats were generated as follows and an effect of suppression of alcohol intake of the compounds of the present invention was evaluated.

Experimental animals used were 6-week-old male Crl:CD(SD) rats and the animals were simultaneously allowed to access 12 v/v% ethanol (containing 5 w/v% sucrose) and water by two-bottle choice between water and ethanol. The animals could access to ethanol and water for a limited period of 2 hours a day and could not access in the rest of 22 hours. The animals had free access to feed for 24 hours. The animals were kept for 3 weeks under the above conditions so that the rats could stably take ethanol. After three weeks of ethanol intake and confirming that the consumption of pure ethanol stably reached 1.5 g/kg/day (as an average of all individuals over 2 to 3 days) or more, an effect of suppression of alcohol intake of test substances was evaluated.

The alcohol-addicted rats were divided into a vehicle administration group and test substance administration groups by using the body weight and pure ethanol consumption as indices according to the random sampling method (Statlight #11). Thirty minutes before starting an access to ethanol and water, test substances A to C each dissolved in saline (containing 5 v/v% dimethylsulfoxide solution) were subcutaneously administered at a dose of 3 mg/kg. To the vehicle administration group, the same volume (5 mL/kg) of 5 v/v% dimethylsulfoxide solution-containing saline was subcutaneously administered.

The ethanol consumption over 2 hours from the start of the access to ethanol and water was measured and the pure ethanol consumption was calculated from the following formula:

$$\text{(Equation) Pure ethanol consumption (Pure EtOH; g/kg)} = A/d_1 \times 0.12 \times d_2/BW$$

A: amount (g) of 12 v/v% ethanol (containing 5 w/v% sucrose) taken
d1: Specific gravity (g/cm$^3$) of 12 v/v% ethanol (containing 5 w/v% sucrose)
d2: Specific gravity of ethanol; 0.79 g/cm$^3$
BW: Rat body weight (kg)

Results

The pure ethanol consumption was significantly lower in the groups to which 3 mg/kg of test substances were administered compared to the vehicle administration group (Figs. 1 to 4). Therefore, it was confirmed that the compound of the present invention exhibited an effect of suppression of ethanol intake in alcohol-addicted rats, and was efficacious for alcohol use disorder.

INDUSTRIAL APPLICABILITY

The medicament containing a compound represented by a general formula (I) or a pharmacologically acceptable salt thereof as an active ingredient is useful as a therapeutic agent for alcohol use disorder.

## Claims

1. A medicament for therapy of alcohol use disorder, comprising a compound represented by a general formula (I) below or a pharmacologically acceptable salt thereof as an active ingredient:

[Chemical Formula 1]

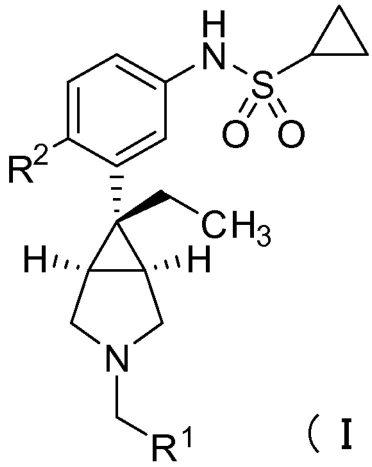

( I )

wherein $R^2$ represents a hydrogen atom or a halogen atom and $R^1$ represents a group selected from the group consisting of

[Chemical Formula 2]

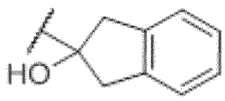

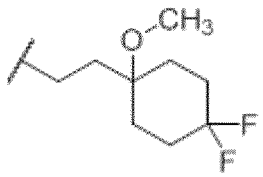

and

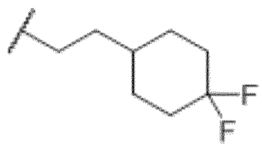

2. The medicament according to claim 1, wherein the active ingredient is a compound or a pharmacologically acceptable salt thereof selected from the group consisting of N-(3- {(1R,5S,6r)-6-ethyl-3-[(2-hydroxy-2,3-dihydro-1H-inden-2-yl)methyl]-3-azabicyclo[3.1.0]hexan-6-yl}-4-fluorophenyl)cyclopropanesulfonamide, N-(3-{(1R,5S,6r)-3-[3-(4,4-difluoro-1-methoxycyclohexyl)propyl]-6-ethyl-3-azabicyclo[3.1.0]hexan-6-yl}phenyl)cyclopropanesulfonamide, and, N-(3-{(1R,5S,6r)-3-[3-(4,4-difluorocyclohexyl)propyl]-6-ethyl-3-azabicyclo [3.1.0]hexan-6-yl}phenyl)cyclopropanesulfonamide.

3. The medicament according to claim 2, wherein the active ingredient is N-(3-{(1R,5S,6r)-6-ethyl-3-[(2-hydroxy-2,3-dihydro-1H-inden-2-yl)methyl]-3-azabicyclo[3.1.0]hexan-6-yl}-4-fluorophenyl)cyclopropanesulfonamide or a pharmacologically acceptable salt thereof.

4. The medicament according to claim 2, wherein the active ingredient is N-(3-{(1R,5S,6r)-3-[3-(4,4-difluoro-1-methoxycyclohexyl)propyl]-6-ethyl-3-azabicyclo[3.1.0]hexan-6-yl}phenyl)cyclopropanesulfonamide or a pharmacologically acceptable salt thereof.

5. The medicament according to claim 2, wherein the active ingredient is N-(3-{(1R,5S,6r)-3-[3-(4,4-difluorocyclohexyl)propyl]-6-ethyl-3-azabicyclo[3.1.0]hexan-6-yl}phenyl)cyclopropanesulfonamide or a pharmacologically acceptable salt thereof.

6. The medicament according to claim 1, wherein the alcohol use disorder is alcoholism.

7. The medicament according to claim 1, wherein the alcohol use disorder is harmful use of alcohol.

8. The medicament according to claim 1, wherein the alcohol use disorder is alcohol abuse.

9. The medicament according to claim 1, wherein the alcohol use disorder is pre-alcoholism.

FIG. 1

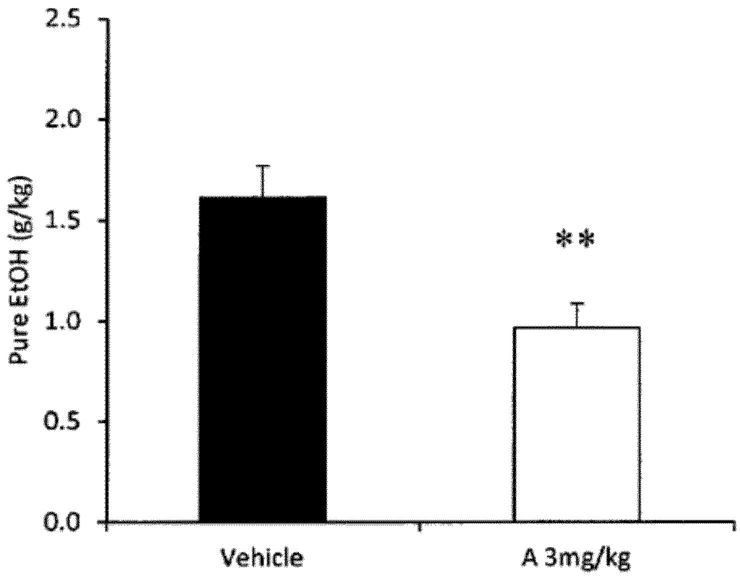


Mean ± S.E. (n=15)

**: P<0.01, when compared with the control group (Student's t-test).

FIG. 2

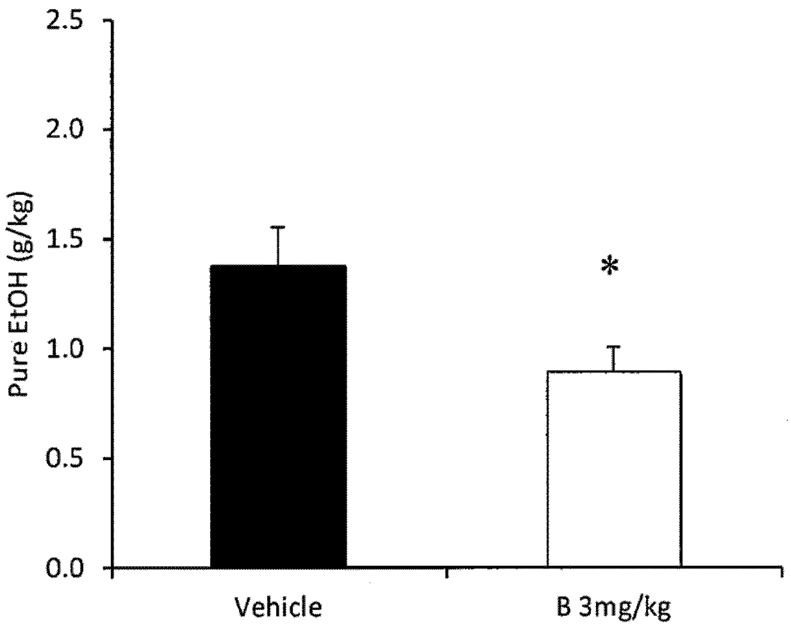


Mean ± S.E. (n=15)

*: P<0.05, when compared with the control group (Student's t-test).

FIG. 3

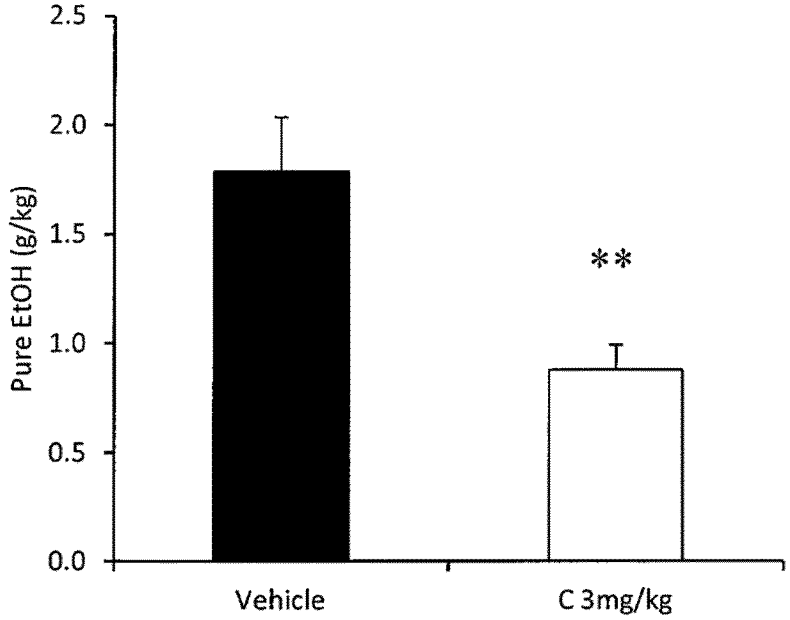


Mean ± S.E. (n=15)

**: P<0.01, when compared with the control group (Aspin-Welch's t-test).

FIG. 4

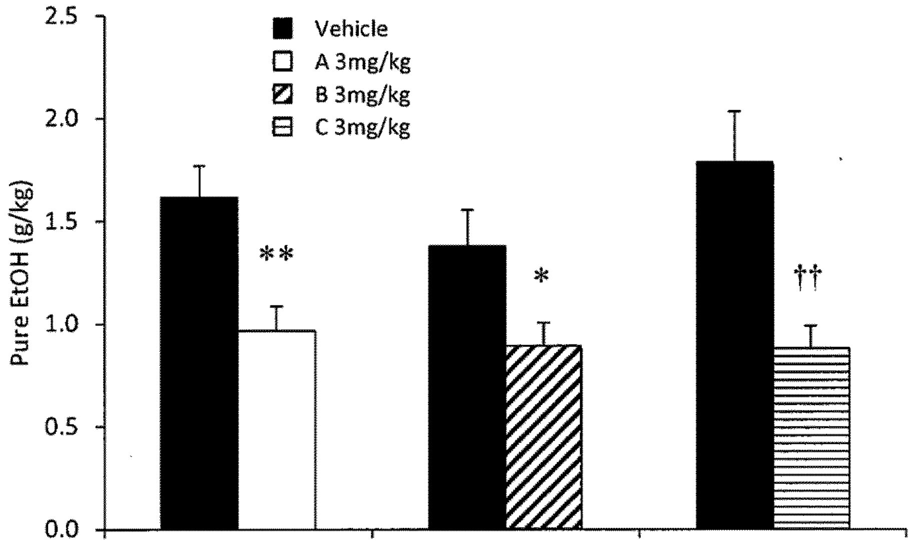


Mean ± S.E. (n=15)

*, **: P<0.05, P<0.01, when compared with the control group (Student's t-test).

††: P<0.01, when compared with the control group (Aspin-Welch's t-test).

## INTERNATIONAL SEARCH REPORT

International application No.
PCT/JP2018/007628

A. CLASSIFICATION OF SUBJECT MATTER
Int.Cl. A61K31/403(2006.01)i, A61P25/32(2006.01)i

According to International Patent Classification (IPC) or to both national classification and IPC

B. FIELDS SEARCHED
Minimum documentation searched (classification system followed by classification symbols)
Int.Cl. A61K31/403, A61P25/32

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| Y | WO 2015/076310 A1 (SANWA KAGAKU KENKYUSHO CO., LTD.) 28 May 2015, claims, examples & US 2016/0280645 A1, claims, examples & EP 3072884 A1 & CN 105764888 A & KR 10-2016-0079789 A | 1-9 |
| Y | JP 2007-502808 A (PFIZER PRODUCTS INC.) 15 February 2007, claims, paragraph [0037] & US 2005/0043327 A1, claims, paragraph [0228] & WO 2005/018645 A1 & EP 1658082 A1 | 1-9 |
| Y | JP 2013-067636 A (RENSSELAER POLYTECHNIC INSTITUTE) 18 April 2013, paragraph [0010] & US 2006/0111384 A1, paragraph [0012] & WO 2006/052710 A1 & EP 1817291 A1 & KR 10-2007-0085723 A & CN 101090891 A | 1-9 |
| Y | JP 2010-537978 A (THERAVANCE, INC.) 09 December 2010, paragraph [0110] & US 7902220 B2, column 19, paragraph [0003] & WO 2009/029256 A1 & EP 2183249 A1 & KR 10-2010-0045524 A & CN 101790529 A | 1-9 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 March 2018 (23.03.2018) | 03 April 2018 (03.04.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2000039089 A
- WO 2001098267 A
- WO 2003035622 A
- WO 2015076310 A


### Non-patent literature cited in the description


- *Drugs,* 1988, vol. 35, 192-213
- *Medicinal Chemistry Communications,* 2011, vol. 2, 1001-1005